# EUROPÄISCHE PATENTSCHRIFT

(11) **EP 1 397 158 B1**
(45) Veröffentlichungstag und Bekanntmachung des Hinweises auf die Patenterteilung: **04.04.2012**
(21) Anmeldenummer: 02747198.6
(22) Anmeldetag: 07.06.2002
(51) Int. Cl.: C07K 16/18, C07K 16/44

(54) **ANWENDUNG VON IgM-ANTIKÖRPERN GEGEN dsDNA BEIM SYSTEMISCHEN LUPUS ERYTHEMATODES MIT NEPHRITIS**
USE OF IgM ANTIBODIES AGAINST dsDNA IN SYSTEMIC LUPUS ERYTHEMATOSUS WITH NEPHRITIS
UTILISATION D'ANTICORPS IgM ANTI-ADN NATIF EN CAS DE LUPUS ERYTHEMATEUX DISSEMINE AVEC NEPHRITE

(30) Priorität: 07.06.2001 DE 10127712
(43) Veröffentlichungstag der Anmeldung: 17.03.2004
(73) Patentinhaber: Witte, Torsten, 30629 Hannover (DE); Matthias, Torsten, 55237 Flonheim (DE)
(72) Erfinder: Witte, Torsten, 30629 Hannover (DE); Matthias, Torsten, 55237 Flonheim (DE)
(74) Vertreter: Fritzsche, Thomas
(86) Internationale Anmeldenummer: PCT/DE2002/002085
(87) Internationale Veröffentlichungsnummer: WO 2002/098459

(56) Entgegenhaltungen:
- EP-A- 0 532 979
- WO-A-91/18628
- WO-A-98/26086
- WO-A-02/096455
- US-A- 4 623 627
- ZOUALI M ET AL: "Suppression of murine lupus autoantibodies to DNA by administration of muramyl dipeptide and syngeneic anti-DNA IgG" JOURNAL OF IMMUNOLOGY, THE WILLIAMS AND WILKINS CO. BALTIMORE, US, Bd. 135, Nr. 2, August 1985 (1985-08), Seiten 1091-1096, XP002218883 ISSN: 0022-1767
- WITTE T ET AL: "IgM anti-dsDNA antibodies in systemic lupus erythematosus: Negative association with nephritis." RHEUMATOLOGY INTERNATIONAL, Bd. 18, Nr. 3, Oktober 1998 (1998-10), Seiten 85-91, XP002244031 ISSN: 0172-8172 in der Anmeldung erwähnt

## Beschreibung

Die Beschreibung offenbart die Verwendung von Immunglobulinen zur Behandlung des systemischen Lupus erythematodes.

Beim Lupus erythematodes (SLE) handelt es sich um eine äußerst schwerwiegende, ernst zu nehmende Erkrankung, die in vielen Fällen zum Tode führt. SLE ist eine Autoimmunerkrankung, bei der Antikörper gegen körpereigene Bestandteile des Zellinneren gebildet werden, die z.B. bei Gewebeläsionen aus der geschlossenen Zelle austreten und vom Immunsystem als körperfremd angesehen und bekämpft werden. Auf diese Weise entsteht ein fataler Reaktionszyklus. SLE tritt vorwiegend erstmals bei jungen Frauen auf. Das Auftreten in der Gesamtbevölkerung beträgt ca. 1:2000, bei Frauen zwischen 20 und 30 Jahren ca. 1:700. Dabei entwickelt sich bei etwa der Hälfte der Patienten im Verlauf der chronischen Erkrankung eine Glomerulonephritis, die früher die häufigste Todesursache des SLE war.

Neben dem charakteristischen sogenannten "Schmetterlings"-Erythem, treten bei lang andauerndem Krankheitsverlauf häufig schwere Gelenkentzündungen auf. Auch rekurierende Pleuritiden mit oder ohne Ergüsse sind häufig. Schwere SLE-Erkrankungen werden üblicherweise mit einer sofortigen Kortikosteroid Therapie behandelt um Folgeschäden, wie eine hämolytische Anämie, Nierenschäden, Vaskulitis sowie akute ZNS-Beteiligungen abzuschwächen. Diese Therapien führen jedoch bei der Schwere dieser lebensbedrohlichen Erkrankung häufig nicht zu befriedigenden Ergebnissen.

Diese generalisierte Autoimmunerkrankung ungeklärter Ätiologie geht üblicherweise mit Bildung zahlreicher Autoantikörper, Immunkomplexen und Veränderungen im Komplementsystem einher. Übliche klinische Beschwerden sind Arthritiden, Hautentzündungen wie Erytheme, Blutbildveränderungen, Nephritiden, Pleuritiden, Perikarditiden und Endokarditiden, wie dem Libmann-Sachs-Syndrom, sowie neurologische und psychische Störungen. Der Krankheitsverlauf ist variabel und endet nach Jahrzehnte langem chronischem Verlauf oft tödlich.

Es ist auch bereits versucht worden diese Erkrankung mit nicht steroidalen Antiphlogistika und Immunsuppressiva sowie mit starken Zytostatika zu behandeln. So beschreiben beispielsweise T. Witte et al. in "Deutsche Medizinische Wochenschrift" 118 (1993), 1005 - 1010, eine Bolustherapie mit Cyclophosphamid zur Behandlung der Lupusnephritis. Die Lupus nephritis ist wegen der Gefahr eines Nierenversagens eine der schwerwiegendsten Organkomplikationen der SLE. Die Cyclophosphamid-Applikation führt jedoch wie alle Zytostatika zu schweren Nebenwirkungen.

H. A. Austin et al., "New Engl. J Med" 1986; 314:614-9 zeigen, dass zur Behandlung der Lupus-Glomerulonephritis die intravenöse Verabreichung von Cyclophosphamid gegenüber der Behandlung mittels Kortikosteroiden, wie Prednison, bezüglich der Überlebensrate überlegen ist. Mittels Cyclophosphamid ist es möglich, bei einem großen Teil der Patienten eine Vollremision bzw. mindestens eine Teilremision zu erreichen.

Um eine möglichst genaue Diagnose des systemischen Lupus erythematodes zu ermöglichen, wurde versucht, eine Korrelation von gängigen Laborparametern und klinischer Manifestation der Erkrankung aufzufinden. So beschreiben T. Witte et al. in "Rheumatol. Int" (1998) 18:85-91, das erhöhte Auftreten von IgM anti-dsDNA-Antikörpern im Serum von SLE-Patienten. Dabei wurden in 52,3 % der Fälle IgM anti-dsDNA-Antikörper gefunden. Dabei zeigte sich, dass zwischen der Entstehung einer Nephritis und Nierenversagen unter IgM anti-dsDNA-Antikörpern eine negative Korrelation besteht, weshalb die Autoren vermuten, dass IgM anti-dsDNA-Antikörper einen Schutz gegen die Entwicklung einer Lupusnephritis anzeigen.

R. Rieben et al. beschreiben in "Blood", Vol. 93, (1999) 942-951, dass die Zugabe von intravenösen Immunglobulin-Präparationen eine Komplementaktivierung blockiert und somit eine antiinflammatorische Wirkung zeigt. Dabei wird Humanserum mit seriellen Verdünnungen von IVIgG sowie IVIgM inkubiert und die Komplementbildung mittels eines ELISA's bestimmt. Dabei zeigte sich eine Dosis-abhängige Inhibierung der Komplementwirkung, weshalb vermutet wird, dass eine intravenöse Verabreichung einer IgM-angereicherten Präparation als Komplementär-Therapie in einer antiinflammatorischen Behandlung verwendet werden könne. Es hat sich jedoch gezeigt, dass die intravenöse Verabreichung von IgM-Antikörpern keinerlei klinische Wirkung zeigt.

In Zouali et al., The Journal of Immunology, vol. 135, No. 2, 1985, pp 1091- 1096, zeigte die subkutane Verabreichung von anti-DNA IgG keinen Effekt auf dem Serumspiegel der anti-DNA Antikörper bei Mänsen, die spontan ein SLE-ähnliches Syndrom entwickeln.

Es ist auch bereits versucht worden, IgM zur Behandlung des Lupus erythematodes intraperitoneal zu applizieren. Auch diese Verabreichung hat keinerlei Wirkung gezeigt (Boes M. et al., Proc. Natl. Acad. Sci. USA (2000) 97:1184-9).

Die Erfindung hat somit zum Ziel, ein Mittel zur Behandlung des systemischen Lupus erythematodes bereitzustellen, welches wirksam ist und die zuvor geschilderten schweren Nebenwirkungen nicht aufweist.

Dieses Ziel wird mit den in den Ansprüchen definierten Merkmalen erreicht.

Es wurde nämlich überraschenderweise gefunden, dass - obwohl sich die intravenöse Verabreichung von IgM-Antikörpern als wirkungslos erwiesen hat - die Verabreichung derartiger Antikörper jedoch dann äußerst wirksam ist, wenn diese subkutan oder intramuskulär appliziert werden. Die Erfindung betrifft somit die Verwendung von Immunglobulinen der M-Klasse zur Herstellung eines Mittels zur Behandlung und/oder Prävention des systemischen Lupus erythematodes zur subkutanen und/oder intramuskulären Applikation. Es werden hierzu solche IgM-Antikörper verwendet, welche gegen körpereigene Antigene gerichtet sind. Körpereigene Antigene sind hier Antigene aus dem Zellinneren, insbesondere dem Zellkern und/oder Cycloplasma, wie Doppelstrang-DNA sowie Phospholipide, insbesondere Kardiolipin.

Es hat sich gezeigt, dass das IgM in beliebiger Form ohne Wirkungsverlust verabreicht werden kann, solange dies s.c. und/oder i.m. geschieht. Üblicherweise wird es jedoch in Form einer Lösung verabreicht, wobei verkapselte Lösungen oder auch verkapselte reine Proteinpulver, insbesondere als Retardformulierung, bevorzugt sind. Die Herstellung derartiger Retardformulierungen ist generell bekannt und umfasst beispielsweise das Einschließen in feine biologisch abbaubare Acrylatkapseln oder das Einlagern in ein biologisch abbaubares dreidimensionales Netzwerk, beispielsweise aus Kollagenfasern, wie es als sogenanntes Catgut bekannt ist. Derartige Depotmatrizes können mittels einer dickeren Nadel ohne weiteres im Unterhautgewebe beispielsweise in einer Bauchfalte deponiert werden. Auf diese Weise ist es möglich, eine über einen beliebigen Zeitraum wirkende Menge an IgM bereitzustellen, welche einen kontinuierlichen Serumspiegel an IgM aufrecht erhält.

Das pharmazeutische Mittel eignet sich insbesondere auch zur Komplementärtherapie zusammen mit anderen bekannten Mitteln zur Behandlung des SLE, und zwar insbesondere antiinflammatorische Mittel wie Aspirin und/oder Kortikosteroiden sowie mit Zytostatika, insbesondere Cyclophosphamid, Azathioprin oder Mycophenolat Mofetil.

Vorgesehen ist auch eine pharmazeutische Kombinationspackung zur gleichzeitigen oder zeitlich versetzten Verabreichung eines erfindungsgemäß hergestellten IgM-enthaltenden Mittels zusammen mit einem weiteren antiinflammatorischen Wirkstoff, einem immunsuppressiven Wirkstoff und/oder einem Zytostatikum. Mit dem therapeutischen Mittel ist es möglich, eine Vielzahl von klinischen Krankheitsbildern des Lupus erythematodes zu behandeln, wie beispielsweise der Nephritis, insbesondere Glomerulonephritis, inklusive einer persistierenden Proteinurie sowie dem akuten und chronischen Nierenversagen sowie anderer Organmanifestationen, wie Schmetterlings-Erythem, Schleimhautulcera, Polyarthritis, insbesondere nicht deformierende Polyarthritis, Arthralgien, Gelenkergüsse, Serositiden, wie Pleuritis und Perikarditis.

Auch die Behandlung mittels Plasmapherese, d.h. ein Plasmaaustausch mittels apparativer Entfernung von Autoantikörpern (Immunapherese), kann mit der Verabreichung des erfindungsgemäß hergestellten Mittels kombiniert werden.

Die Erfindung soll an dem folgenden Beispiel näher erläutert werden.

Es wurden sogenannte NZB x NZW (B/W)-Mäuse als Modellsystem verwendet. Solche Mäuse sind eines der Standard-Tiermodelle des SLE, da sie typische Autoantikörper und nach ca. 6-8 Monaten spontan eine Lupusnephritis entwickeln. Es wurden daher vier Behandlungsgruppen mit jeweils 10 Mäusen gebildet. Diese Mäuse erhielten folgende Therapie:
A: Kontrollgruppe, erhielt keine Therapie
B: Diese Gruppe erhielt jeweils intravenös 100 µg eines monoklonalen HB 8329 Antikörpers ab einem Alter von 4 Monaten in zweiwöchentlichen Abständen.
C: Diese Gruppe erhielt eine subkutane Injektion von jeweils 100 µg des monoklonalen Antikörpers HB 8329 ab einem Alter von vier Monaten in wöchentlichen Abständen.
D: Diese Gruppe erhielt eine IV-Injektion von je 100 µg des monoklonalen Antikörpers HB 8329 ab einem Alter von sieben Monaten, d.h. nach Beginn der Nephritis in zweiwöchentlichen Abständen.

Der Antikörper wurde aus dem Kulturüberstand des Hybridoms HB 8329 (ATCC) isoliert und wie folgt gereinigt:

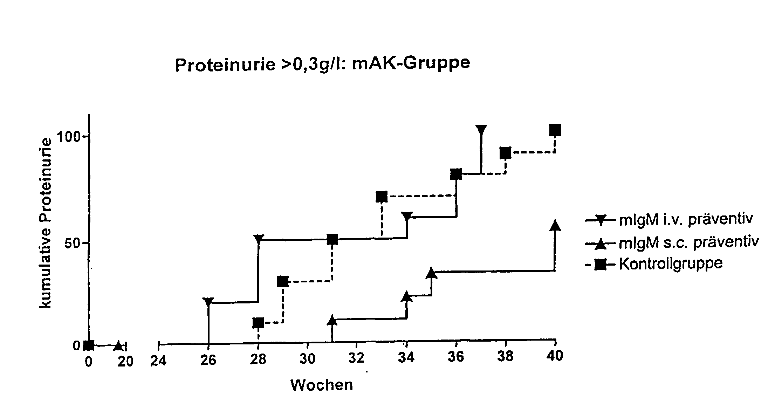

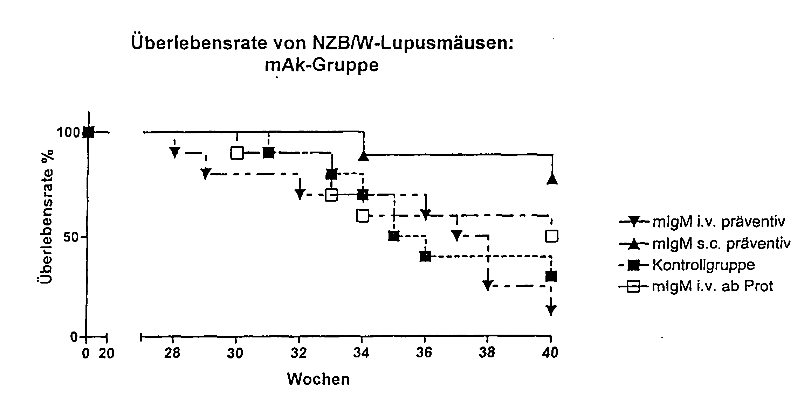

Das Hybridom HB 8329 wurde in RPMI 1640 mit einem Zusatz von Penicillin/Streptomycin und ITS-Medium ohne FCS kultiviert. Der Kulturüberstand wird auf einer Supertex 200 Säule (Pharmacia & Upjohn) aufgetragen und das große IgM-Molekül von allen anderen Bestandteilen des Kulturmediums abgetrennt. Die Ergebnisse sind in Abbildung 1 und Abbildung 2 dargestellt.

Hierbei zeigen
- Abb. 1: die Zunahme bzw. die Entstehung der Proteinurie und
- Abb. 2: die Überlebensrate der Versuchstiere.

Wie Abb. 1 zu entnehmen ist, entspricht der Verlauf der kumulativen Proteinurie bei präventiver intravenöser Verabreichung von Immunglobulin M etwa dem der unbehandelten Kontrollgruppe. Dies bedeutet, dass die intravenöse Verabreichung von Immunglobulin M keinerlei Schutzwirkung zeigt. Im Gegensatz dazu zeigt die subkutane Applikation von IgM einen deutlich späteren Ausbruch der Erkrankung sowie einen deutlich langsameren Verlauf.

In Abb. 2 wird gezeigt, dass die intravenöse Verabreichung ab entstehender Proteinurie ebenso wie die intravenöse präventive Verabreichung im Rahmen der statistischen Verteilung keinerlei Wirkung auf das Überleben der Mäuse zeigt. Hierbei liegt die Todesrate nach 40 Wochen in der Kontrollgruppe sowohl bei der präventiven als auch akuten intravenösen Applikation bei 20 - 50 %, wohingegen die Überlebensrate bei der subkutanen präventiven Applikation bei ca. 80 - 85 % liegt. Auch dies belegt die überraschende und überragende Wirkung der erfindungsgemäßen Verwendung von Immunglobulinen der M-Klasse.

## Patentansprüche

1. Verwendung von Immunglobulin M zur Herstellung eines Mittels zur Behandlung und/oder Prävention des systemischen Lupus erythematodes zur subkutanen und/oder intramuskulären Applikation, wobei das IgM gegen Autoantigene des Zellkerns des Cytoplasmas sowie der Phospholipide gerichtet ist.

2. Verwendung nach Anspruch 1, **dadurch gekennzeichnet, dass** das IgM ein Anti-dsDNA ist.

3. Verwendung nach Anspruch 1, **dadurch gekennzeichnet, dass** das IgM ein Anti-Kardiolipin-IgM ist.

4. Verwendung nach einem der vorhergehenden Ansprüche, **dadurch gekennzeichnet, dass** das Mittel IgM in Lösung, verkapselt, als Retardformulierung oder als Reinsubstanz enthält.

5. Verwendung nach einem der vorhergehenden Ansprüche, **dadurch gekennzeichnet, dass** das Mittel in ein aus abbaubaren Kollagenfasern eingeschlossenes Netzwerk eingelagert ist.

6. Verwendung nach einem der vorhergehenden Ansprüche,
**dadurch gekennzeichnet, dass** das IgM in eine Mischung von Netzwerken eingeschlossen ist, welche eine unterschiedliche Auflösungsgeschwindigkeit aufweisen.

7. Pharmazeutische Kombination zur Behandlung und/oder Prävention des systemischen Lupus erythematodes enthaltend ein IgM, welches gegen Autoantigene des Zellkerns und/oder der Phospholipide gerichtet ist, sowie mindestens ein antiinflammatorisches Mittel, mindestens ein immunsuppressives Mittel und/oder mindestens ein Zytostatikum zur gleichzeitigen und/oder zeitlich versetzten Applikation.

8. Pharmazeutische Kombination nach Anspruch 7, **dadurch gekennzeichnet, dass** das antiinflammatorische Mittel Acetylsalicylsäure und/oder ein Kortikosteroid ist.

9. Pharmazeutische Kombination nach Anspruch 7 oder 8, **dadurch gekennzeichnet, dass** das zytostatische Mittel Cyclophosphamid und/oder Azathioprin, Methotrexat und/oder Mycophenolat-Mofetil ist.

10. Pharmazeutische Kombination nach einem der Ansprüche 7 - 9, **dadurch gekennzeichnet, dass** das immunsuppressive Mittel Cyclosporin, Leflunomid und/oder ein mono- oder polyklonaler Antilymphozyten-Antikörper ist.

## Claims

1. Use of immunoglobulin M for the manufacture of a medicament for treating or preventing systemic lupus erythematosus for sub-cutaneous and/or intra-muscular application, wherein the IgM is directed against autoantigens of the cell nucleus of the cytoplasm and of the phospholipids.

2. Use according to claim 1, **characterised in that** the IgM is an anti-dsDNA.

3. Use according to claim 1, **characterised in that** the IgM is an anti-cardiolipin IgM.

4. Use according to any one of the preceding claims, **characterised in that** the means contains IgM in solution, encapsulated, as a controlled-release formulation or as a pure substance.

5. Use according to any one of the preceding claims, **characterised in that** the means is embedded in a network enclosed by degradable collagen fibres.

6. Use according to any one of the preceding claims, **characterised in that** the IgM is enclosed in a mix of networks having different dissolution rates.

7. Pharmaceutical combination for treating or preventing systemic lupus erythematosus containing an IgM that is directed against autoantigens of the cell nucleus and/or of the phospholipids, and at least one anti-inflammatory agent, at least one immuno-suppressive agent and/or at least one cytostatic agent for the simultaneous and/or chronologically staggered application.

8. Pharmaceutical combination, according to claim 7, **characterised in that** the anti-inflammatory agent is acetylsalicylic acid and/or a corticosteroid.

9. Pharmaceutical combination according to claim 7 or 8, **characterised in that** the cytostatic agent is cyclophosphamide and/or azathioprine, methotrexate and/or mycophenolate mofetil.

10. Pharmaceutical combination according to one of the claims 7-9, **characterised in that** the immuno-suppressive agent is cyclosporin, leflunomide and/or a mono- or polyclonal antilymphocyte antibody.

## Revendications

1. Utilisation d'immunoglobuline M pour la préparation d'un médicament destiné à traiter et/ou à prévenir le lupus érythémateux systémique pour une administration sous-cutanée et/ou intramusculaire, l'IgM étant dirigée contre l'autoantigène du noyau cellulaire du cytoplasme, ainsi que des phospholipides.

2. Utilisation selon la revendication 1, **caractérisée en ce que** l'IgM est un anti-ADNdb.

3. Utilisation selon la revendication 1, **caractérisée en ce que** l'IgM est un anti-cardiolipine IgM.

4. Utilisation selon l'une quelconque des revendications précédentes, **caractérisée en ce que** le médicament contient l'IgM en solution, encapsulée, en tant que formulation retard ou en tant que substance pure.

5. Utilisation selon l'une quelconque des revendications précédentes, **caractérisée en ce que** le médicament est entreposé dans un réseau enfermé de fibres collagènes dégradables.

6. Utilisation selon l'une quelconque des revendications précédentes, **caractérisée en ce que** l'IgM est enfermée dans un mélange de réseaux, lesquels présentent une vitesse de dissolution variable.

7. Combinaison pharmaceutique destinée au traitement et/ou à la prévention du lupus érythémateux systémique comprenant une IgM, laquelle est dirigée contre les autoantigènes du noyau cellulaire et/ou des phospholipides, ainsi qu'au moins un agent anti-inflammatoire, au moins un agent immunosuppresseur et/ou au moins un cytostatique pour une administration simultanée et/ou décalée dans le temps.

8. Combinaison pharmaceutique selon la revendication 7, **caractérisée en ce que** l'agent anti-inflammatoire est un acide acétylsalicylique et/ou un corticostéroïde.

9. Combinaison pharmaceutique selon la revendication 7 ou 8, **caractérisée en ce que** le médicament cytostatique est le cyclophosphamide et/ou l'azathioprine, le méthotrexate et/ou le mycophénolate mofétil.

10. Combinaison pharmaceutique selon l'une quelconque des revendications 7 à 9, **caractérisée en ce que** l'agent immunosuppresseur est la ciclosporine, le léflunomide et/ou un anticorps antilymphocytes mono- ou polyclonaux.
